# EUROPÄISCHE PATENTSCHRIFT

(11) **EP 1 615 598 B1**
(45) Veröffentlichungstag und Bekanntmachung des Hinweises auf die Patenterteilung: **07.05.2008**
(21) Anmeldenummer: 04729023.4
(22) Anmeldetag: 23.04.2004
(51) Int. Cl.: A61F 2/44, A61F 2/46

(54) **VORRICHTUNG FÜR DIE SPONDYLODESE**
SPONDYLODESIS DEVICE
DISPOSITIF DE SPONDYLODESE

(30) Priorität: 23.04.2003 EP 03405284
(43) Veröffentlichungstag der Anmeldung: 18.01.2006
(73) Patentinhaber: Sepitec Foundation, 9490 Vaduz (LI)
(72) Erfinder: MAGERL, Friedrich, CH-9011 St. Gallen (CH); STADLER, Roger, CH-8008 Zürich (CH); WIDMER, Christian, CH-9430 St. Margrethen (CH)
(74) Vertreter: Groner, Manfred
(86) Internationale Anmeldenummer: PCT/CH2004/000244
(87) Internationale Veröffentlichungsnummer: WO 2004/093749

(56) Entgegenhaltungen:
- WO-A-00/22998
- GB-A- 1 243 353
- US-A- 5 360 430
- US-A- 5 443 515
- US-A- 5 755 796
- US-A- 6 156 037
- US-A1- 2001 007 072
- US-B1- 6 306 136

## Beschreibung

Die Erfindung betrifft eine Vorrichtung für die Spondylodese nach dem Oberbegriff des Anspruchs 1.

Als Spondylodese wird die chirurgische Versteifung oder Verblockung eines Abschnittes der Wirbelsäule verstanden. Hierzu wird nach der klassischen Methode Knochen- oder Knochenersatzmaterial verwendet, welches zwischen die Wirbelkörper eingesetzt wird, sogenannte interkorporelle Spondylodese, oder über die hinteren Wirbelelemente angelagert wird, sogenannte dorsale Spondylodese. Hierdurch entsteht mit der Zeit eine knöcherne Brücke, welche die Wirbel verbindet, so dass sie sich nicht mehr gegeneinander bewegen können. Damit eine Verknöcherung stattfinden kann, ist es erforderlich, dass der betreffende Abschnitt der Wirbelsäule ruhiggestellt wird. Letzteres geschieht durch geeignete Implantate. Diese können mit dem Intervertebralimplantat verbunden oder von diesem unabhängig angebracht werden.

Mit konservativen Behandlungsmassnahmen nicht beeinflussbare Schmerzen, Rückenmark- oder Nervenwurzelkompressionen sowie Fehlstellungen sind Indikationen für Spondylodesen. Schmerzen können prinzipiell von allen krankhaft veränderten Strukturen der Wirbelsäule ausgehen. Für die Entwicklung von Rückenmark- oder Nervenwurzelkompressionen sind Verengungen des Wirbelkanals oder der Zwischenwirbellöcher verantwortlich. Mit einem chirurgischen Eingriff werden die pathologischen Veränderungen beseitigt und die Stabilität der Wirbelsäule durch die Spondylodese wieder hergestellt.

Weil bei der interkorporellen Spondylodese immer Bandscheiben ausgeräumt werden und dies die Stabilität der Wirbelsäule beeinträchtigt, ist diese immer wieder herzustellen. Dies kann beispielsweise mit druckfesten Knochenspänen geschehen, die man vom Patienten entnimmt, sogenannte autogene Knochenspäne, und zwischen den Wirbelkörpern einsetzt. Da die Belastbarkeit solcher Späne oft unsicher und ihre Verfügbarkeit begrenzt ist, und zudem die durch eine Spanentnahme verursachte Morbidität erheblich sein kann, benutzt man anstelle von autogenen Knochenspänen zunehmend aus körperfremden Materialien hergestellte Intervertebralimplantate, auch "Cages" genannt.

Ein Intervertebralimplantat funktioniert als druckaufnehmender Platzhalter, welcher die Spondylodese stabilisiert, die Einstellung der Wirbelkörper zueinander sichert und gewährleistet, dass sich zwischen den benachbarten Wirbelkörpern eine solide Knochenbrücke bildet. In die Intervertebralimplantate eingefülltes und/oder um sie herum angelagertes Knochen- oder Knochenersatzmaterial bilden eine Matrix für eine Knochenneubildung. Für den Ossifikationsprozess spielt die Stabilität der Spondylodese eine entscheidende Rolle. Innerhalb der Spondylodese stattfindende Bewegungen verzögern oder verhindern deren knöcherne Konsolidierung.

Im Stand der Technik sind zahlreiche Intervertebralimplantate bekannt geworden, die mit einer Platte mit Wirbeln verbunden werden.

Die gattungsbildende US-A-5,360,430 offenbart eine Vorrichtung, die zwei Platten aufweist, die jeweils mit einem Wirbelkörper verbindbar sind und mit einem Implantat jeweils ein Schiebegelenk bilden, das mit Spannschrauben fixierbar ist. Das Implantat dient als Wirbelersatz und besitzt ein Kugelgelenk oder eine Spiralfeder.

Die US-A-5,443,515 betrifft ebenfalls eine Vorrichtung, mit welcher ein beschädigter Wirbelkörper ersetzt werden kann.

Die US 6,235,059 B offenbart eine Vorrichtung mit einem Intervertebralimplantat, mit dem zwei Wirbel aneinander stabilisiert werden können. Am Intervertebralimplantat ist eine zweiarmige Platte befestigt, die starr oder schwenkbar mit dem Intervertebralimplantat verbunden ist. Jeder Arm besitzt eine Durchgangsbohrung, durch die eine Knochenschraube in den entsprechenden Wirbelkörper einschraubbar ist. Eine Anpassung an die jeweiligen anatomischen Gegebenheiten und insbesondere an die sagittale Krümmung ist hier sehr beschränkt. Das Intervertebralimplantat kann nur mit den benachbarten Wirbeln verbunden werden.

Die WO 00/24343 offenbart eine Vorrichtung mit einem Intervertebralimplantat, das am Umfang Windungen besitzt und ähnlich einer Schraube zwischen die Wirbel eingeschraubt werden kann. An einem vorderen Ende des Intervertebralimplantates ist eine flügelförmige zweiarmige Platte befestigt. Die Befestigung ist hier so ausgeführt, dass die Platte zur Anpassung an die anatomischen Gegebenheiten bewegbar ist. Die beiden Arme der Platte weisen jeweils ein Langloch für die Aufnahme einer Knochenschraube auf. Auch bei dieser Vorrichtung kann das Intervertebralimplantat nur mit den benachbarten Wirbeln verbunden werden. Die Anpassbarkeit an die anatomischen Gegebenheiten ist sehr beschränkt.

Die FR 2 727 005 offenbart eine Vorrichtung mit mehreren Intervertebralimplantaten, die mit einem gemeinsamen Band miteinander verbunden werden. Das Band weist Löcher zur Befestigung des Bandes und jeweils ein Intervertebralimplantat und Löcher zur Aufnahme einer Knochenschraube auf. Mit dieser Vorrichtung können zwar mehrere Intervertebralimplantate miteinander verbunden werden, die Anpassbarkeit an die anatomischen Gegebenheiten ist auch hier jedoch sehr beschränkt. Um eine solche Anpassbarkeit zu gewährleisten, müssten hier entsprechend unterschiedliche änder zur Verfügung stehen. Die Abstände zwischen den Intervertebralimplantaten ist vorgegeben.

Die EP 0 179 695 A offenbart ein Intervertebralimplantat, an dem Ösen angeformt sind, mit denen das Implantat an benachbarten Wirbeln festschraubbar ist. Auch hier ist die Anpassbarkeit an anatomische Gegebenheiten sehr beschränkt. Insbesondere ist es hier in der Regel nicht möglich, die Knochenschrauben an der geeigneten Stelle in den Wirbelkörper einzusetzen. Es können auch hier nur zwei Wirbel mit einem Intervertebralimplantat verbunden werden.

Die US 5,360,430 offenbart ein Intervertebralimplantat, welches einen Wirbel ganz oder teilweise ersetzt, welches mit Platten an den oberen resp. unteren Wirbeln befestigt wird und welches zwischen diesen Wirbeln eine gewisse ungefederte oder gefederte Beweglichkeit erlaubt.

Die S 5,443,515 offenbart ebenfalls ein Implantat, welches einen Wirbelkörper ersetzen soll. Es ist aus Tantalschaum hergestellt.

Die Stabilisierung von Intervertebralimplantaten mit herkömmlichen Platten erfordert somit wegen der grossen individuellen und krankheitsbedingten anatomischen Variabilität insbesondere der Halswirbelsäule ein grosses Sortiment verschieden langer Platten mit unterschiedlichen Lochabständen. Die Länge der Platten richtet sich dabei nach der Position der oberen und unteren Endlöcher. Wenn bei mehrsegmentalen Spondylodesen die Platten nur an die Endwirbel befestigt werden, besteht die Gefahr, dass die Platten an einem Endwirbel ausreissen. Es ist deshalb ratsam, die Platten auch an die zwischen den Endwirbeln liegenden Wirbelkörper mit Schrauben zu fixieren. Die bei herkömmlichen Plat ten dafür vorgesehenen Schraubenlöcher liegen aber nicht immer genau so, dass die betreffenden Schrauben gut in die zwischen den Endwirbeln liegenden Wirbelkörper eingesetzt werden können. Zudem kann bei verschiedenen Plattentypen nur jeweils eine Schraube im Wirbelkörper befestigt werden.

Der Erfindung liegt die Aufgabe zugrunde, eine Vorrichtung der genannten Art zu schaffen, die optimaler an die jeweiligen anatomischen Gegebenheiten anpassbar ist.

Die Aufgabe ist bei einer gattungsgemässen Vorrichtung gemäss Anspruch 1 gelöst. Ein wesentlicher Vorteil der erfindungsgemässen Vorrichtung besteht darin, dass auch Mehretagenspondylodesen hergestellt werden können, wobei benachbarte Intervertebralimplantate direkt miteinander verbunden werden können. Dadurch ergibt sich eine besonders hohe Stabilität und damit Sicherheit. Die Intervertebralimplantate bilden somit mit den Platten eine besonders stabile Einheit. Es können auch unterschiedlich grosse Intervertebralimplantate kombiniert und mit unterschiedlichen Platten untereinander winkelstabil verbunden werden. Insbesondere ist eine Anpassung an unterschiedliche Krümmungen der Wirbelsäule und an unterschiedliche Abstände zwischen Intervertebralimplantaten und Wirbeln möglich. Die Erfindung eignet sich insbesondere für Platten, die plastisch nicht deformierbar sind, und beispielsweise aus Titan oder einem Verbundwerkstoff bestehen.

Die Vorrichtung eignet sich insbesondere für die Spondylodese an der Halswirbelsäule. Hier empfiehlt es sich, die Spondylodese mit an die Vorderfläche der Halswirbelkörper fixierten Platten abzusichern. Es ist insbesondere ein Modularsystem möglich, das sowohl in der Herstellung als auch in der Praxis vorteilhaft ist. Die Anpassbarkeit ist mit einem überraschend kleinen Sortiment aus Einzelteilen möglich.

Nach einer Weiterbildung der Erfindung ist vorgesehen, dass wenigstens eine Platte Z-förmig oder L-förmig ausgebildet ist. Mit solchen Platten ist eine besonders hohe Anpassbarkeit und ein besonders stabiler Verbund möglich. Z-förmige Platten eignen sich insbesondere zum Verbinden benachbarter Intervertebralimplantate und damit zur Stabilisierung von drei Wirbeln. Solche Z-förmige Platten können in einem mittleren Bereich beispielsweise mit zwei Knochenschrauben verankert werden. L-förmige Platten ermöglichen ebenfalls eine Befestigung solcher Platten mit zwei Knochenschrauben. Auf dieselbe Art können auch mehr als drei Wirbel resp. mehr als zwei Intervertebralimplantate untereinander verbunden werden. Als Implantatbrücken eignen sich auch gerade Platten, welche durch fixierbare Gelenke an die Intervertebralimplantate und Knochenschrauben an den zwischen Intervertebralimplantaten liegenden Wirbelkörper befestigt werden. Denkbar ist auch eine Ausführung, bei welcher zwei Intervertebralimplantate nicht direkt durch eine sie verbindende Platte (Implantatbrücke) aneinander fixiert werden, sondern indirekt über den zwischen den beiden Intervertebralimplantaten liegenden Wirbelkörper. Für diesen Fall sind kurze Platten vorgesehen, welche durch fixierbare Gelenke an das jeweilige Intervertebralimplantat und mit Knochenschrauben an den Wirbelkörper befestigt werden, wobei dann die am oberen Intervertebralimplantat angebrachte Platte nach unten und die am unteren Intervertebralimplantat angebrachte Platte nach oben gerichtet ist, sodass die Plattenenden mit den Knochenschrauben am Zwischenwirbel nebeneinander liegen. Somit ist dann jedes der beiden Intervertebralimplantate mit durch eine Platte und mindestens eine Knochenschraube mit dem Zwischenwirbel verbunden.

Die Erfindung betrifft auch einen Bausatz zur Herstellung einer Vorrichtung gemäss Anspruch 1.

Ausführungsbeispiele der Erfindung werden nachfolgend anhand der Zeichnungen näher erläutert. Es zeigen:
- Fig. 1: schematisch eine räumliche Ansicht einer erfindungsgemässen Vorrichtung,
- Fig. 2: eine weitere Ansicht der erfindungsgemässen Vorrichtung gemäss Fig. 1,
- Fig. 3: einen Schnitt entlang der Linie III - III der Fig. 2,
- Fig. 4: eine weitere Ansicht der erfindungsgemässen Vorrichtung gemäss Fig. 1,
- Fig. 5: eine weitere Ansicht der erfindungsgemässen Vorrichtung gemäss Fig. 1,
- Fig. 6: eine räumliche Ansicht einer Variante der erfindungsgemässen Vorrichtung,
- Fig. 7: eine räumliche Ansicht einer Variante der erfindungsgemässen Vorrichtung,
- Fig. 8: eine räumliche Ansicht einer geraden Platte mit einem Gelenk,
- Fig. 9: eine räumliche Ansicht einer geraden Platte mit zwei Gelenken, und
- Fig. 10-15: schematisch Ansichten eines Abschnittes einer Wirbelsäule mit jeweils einer erfindungsgemässen Vorrichtung, welche Wirbel miteinander verbindet.

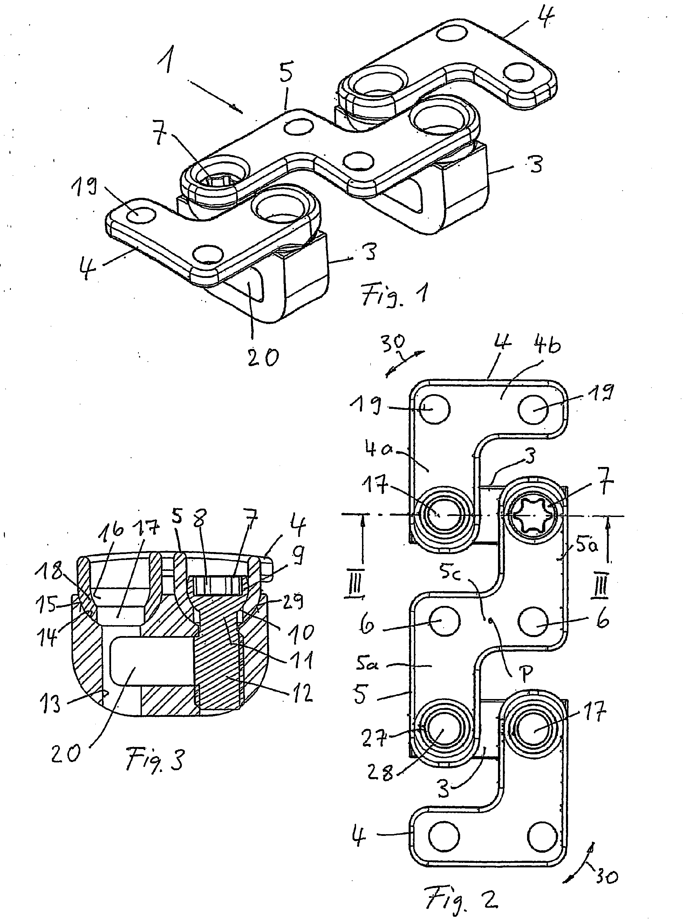

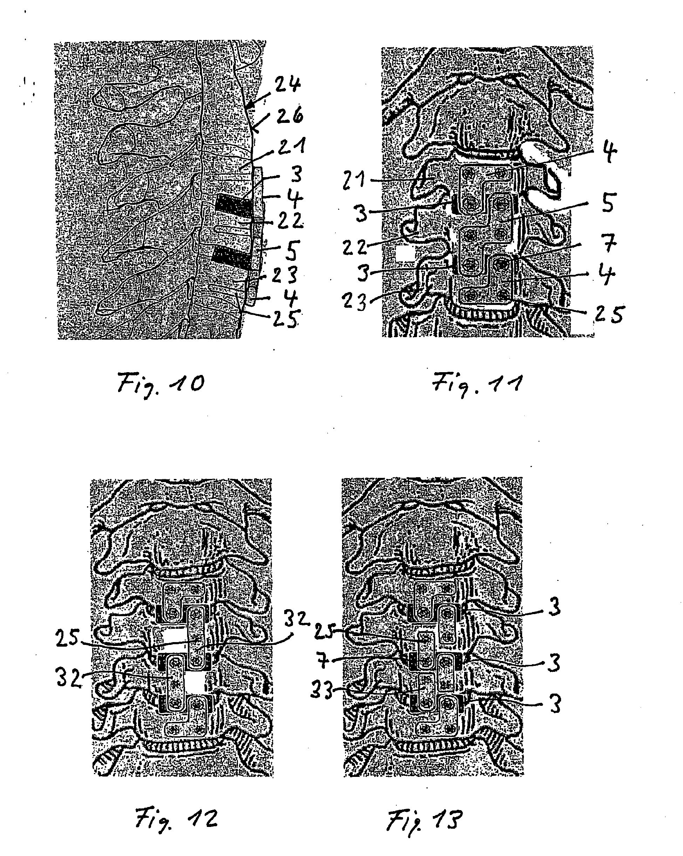

Die in den Fig. 1 bis 4 gezeigte Vorrichtung 1 weist zwei Intervertebralimplantate 3 auf, die gemäss den Fig. 10 und 11 jeweils in den Bandscheibenraum benachbarter Wirbel 21 und 22 bzw. 22 und 23 einer Wirbelsäule 24 eingesetzt sind. Die Wirbel 21 bis 23 sind hier insbesondere Wirbel der Halswirbelsäule, wobei mit 26 die Vorderfläche der Halswirbelsäule bezeichnet ist. In der

Fig. 10 liegen links vor der Vorderfläche hier nicht gezeigte Speiseröhre und grosse Blutgefässe.

Die beiden Intervertebralimplantate 3 bestehen aus Metall, insbesondere Titan oder einem geeigneten Kunststoff und sind interkorporelle und druckaufnehmende Körper. Sie weisen jeweils eine nach aussen offene Ausnehmung 20 auf, die zur Aufnahme von Knochen oder Knochenersatzmaterial dient. Die Ausnehmungen 20 werden vom Knochen des Patienten durchwachsen, so dass sich eine knöcherne Brücke zwischen den benachbarten Wirbeln 21 und 22 bzw. 22 und 23 bildet. Die Wirbel 21, 22 und 23 können damit fest miteinander verbunden werden.

Die beiden Intervertebralimplantate 3 sind mit einer Z-förmigen Platte 5 fest miteinander verbunden. Diese Platte 5 weist gemäss Fig. 2 zwei Arme 5a und 5b sowie einen quer zu diesen verlaufenden mittleren Bereich 5c auf. Die beiden Arme 5a und 5b sind vorzugsweise gleich ausgebildet, so dass die Platte 5 bezüglich eines mittleren Punktes P drehsymmetrisch ist. Die beiden Arme 5a weisen jeweils an einem freien Ende eine halbkugelförmige Vertiefung 27 auf, durch die mittig eine Durchgangsöffnung 17 hindurch geht. Diese halbkugelförmigen Vertiefungen 27 und die Durchgangsöffnungen 17 nehmen jeweils eine Kugelkopfschraube 7 auf, die mit einem Gewindeschaft 12 in eine Bohrung 13 eines Intervertebralimplantates 3 eingeschraubt ist. Die Kugelkopfschrauben 7 weisen jeweils einen Kopf 9 mit einem Werkzeugangriff 8 und einer ebenfalls halbkugelförmigen Unterseite 10 auf. Zwischen dem Schaft 12 und dem Kopf 9 ist ein Hals 11 angeordnet, dessen Durchmesser kleiner ist als derjenige des Schaftes 12 und der Durchgangsöffnung 28. Die halbkugelförmige Unterseite 10 liegt an der halbkugelförmigen Vertiefung 27 an. Sind die beiden Schrauben 12 fest in das jeweilige Intervertebralimplantat 3 eingeschraubt, so ist die Platte 5 an ihren beiden freien Enden fest am jeweiligen Intervertebralimplantat 3 durch Klemmung fixiert. Die Klemmflächen werden durch eine halbkugelförmige Fläche 15 und eine ebenfalls halbkugelförmige Fläche 14 des entsprechenden Intervertebralimplantates 3 gebildet. Sind die beiden Kugelkopfschrauben 7 nicht vollständig eingeschraubt, so bilden die beiden Verbindungen der Platte 5 zu den Intervertebralimplantaten 3 jeweils eine Art Kugelgelenk, die jeweils an beiden Befestigungspunkten Bewegungen in mehreren Freiheitsgraden ermöglichen.

Die Platte 5 und die beiden Intervertebralimplantate 3 können somit gegeneinander bewegt werden und in jeder gewünschten Position können diese Teile durch Festziehen der Schrauben 7 winkelstabil miteinander verbunden werden. Nach dem Festziehen der Kugelkopfschrauben 7 bilden die beiden Intervertebralimplantate 3 und die Z-förmige Platte 5 eine stabile Einheit. Nötigenfalls können die Schrauben 7 jederzeit gelöst und damit wieder der bewegliche Zustand hergestellt werden.

An den beiden Intervertebralimplantaten 3 ist jeweils eine weitere Platte 4 angeordnet, die vorzugsweise L- bzw. winkelförmig ausgebildet sind. Diese Platten 4 weisen jeweils zwei Arme 4a und 4b auf, die gleich oder unterschiedlich sein können. Der Arm 4a weist an seiner Unterseite einen kugelförmigen Gelenkteil 18 mit einer kugelförmigen Innenfläche 16 und einer ebenfalls halbkugelförmigen Aussenfläche 15 auf. Der Gelenkteil 18 weist eine Durchgangsöffnung 17 auf. Der Gelenkteil 18 sitzt in einer halbkugelförmigen Vertiefung 14 des entsprechenden Intervertebralimplantates 3. Um die Platte 4 am entsprechenden Intervertebralimplantat 3 zu befestigen, wird gleich wie zur Befestigung der Z-förmigen Platte 5 in den Gelenkteil 18 von oben eine Kugelkopfschraube 7 eingesetzt und in die Bohrung 13 des entsprechenden Intervertebralimplantates 3 eingeschraubt. In den Fig. 1 - 3 sind diese Schrauben 7 aus zeichnerischen Gründen nicht eingezeichnet. Vor dem Festziehen der Schraube 7 kann die entsprechende, Platte 4 aufgrund der kugelgelenkartigen Verbindung bewegt und an die anatomischen Gegebenheiten angepasst werden. Ist die optimale Position erreicht, so werden die entsprechenden Schrauben 7 angezogen und damit die Platten 4 winkelstabil bezüglich des entsprechenden Intervertebralimplantates 3 fixiert.

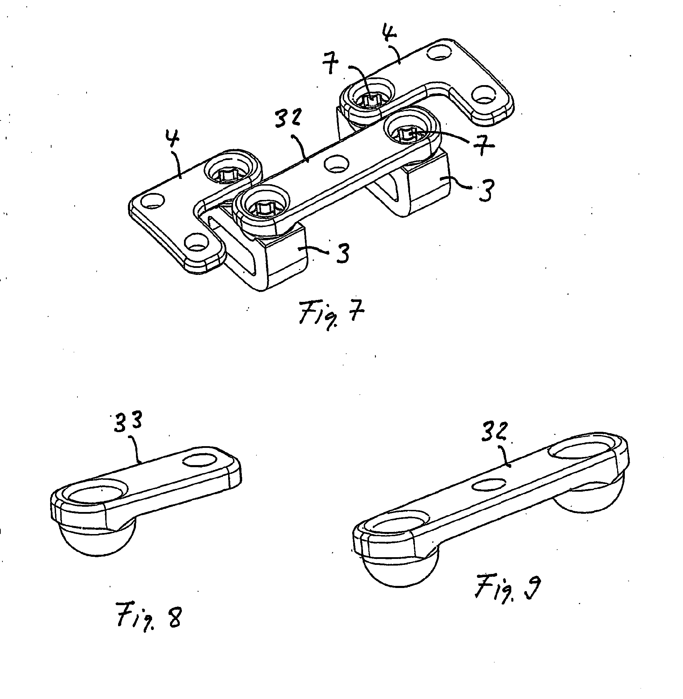

Die beiden Platten 4 weisen im Arm 4b jeweils zwei im Abstand zueinander angeordnete Durchgangsöffnung 19 auf, die jeweils zur Aufnahme einer Knochenschraube 25 dienen, wie dies in Fig. 7 schematisch dargestellt ist. Solche Knochenschrauben 25 sind an sich bekannt. Die Verbindung kann mit einer Schraube gemäss der WO 01/30251 winkelstabil oder alternativ auch beweglich sein. Mit diesen Schrauben 25 werden die beiden Platten 4 im entsprechenden Wirbelkörper 21 bzw. 23 befestigt. Diese Knochenschrauben 25 sollten möglichst in der Mitte des entsprechenden Wirbelkörpers 21 bzw. 23 liegen. Andernfalls könnte ihr Halt beeinträchtigt werden. Diese Schrauben sollten ferner nicht in eine gesunde Bandscheibe eindringen und zudem sollten die Arme 4b nicht eine gesunde Bandscheibe berühren. Dies würde zu Zerstörungen bzw. Degenerationen der betreffenden Bandscheiben führen. Aufgrund der Einstellbarkeit der Platten 4 ist es nun möglich, diese so auszurichten, dass die Schrauben wie oben erläutert in geeigneten Positionen in die Wirbelkörper 21 bzw. 23 eingeschraubt werden können. Ebenfalls kann damit vermieden werden, dass die Arme 4b eine Bandscheibe berühren.

Die genannte Einstellbarkeit der Platten 4 lassen sich diese an die unterschiedlichen Krümmungen und Formen der Vorderfläche 26 der Halswirbelsäule anpassen. Dies ist auch dann möglich, wenn Intervertebralimplantate 3 wie gewünscht nicht an der Vorderseite 26 vorstehen. Die Intervertebralimplantate 3 dürfen an der Vorderfläche 26 nicht vorstehen, da sie dadurch Verletzungen der vor der Halswirbelsäule 24 angeordneter Gebilde und insbesondere der Speiseröhre und grosser Blutgefässe verursachen könnten.

Die beiden Platten 4 verbinden somit bei festgezogenen Kugelkopfschrauben 7 jeweils ein Intervertebralimplantat 3 winkelstabil mit einem Wirbelkörper 21 und 22 bzw. 22 und 23. Die Platte 5 verbindet die beiden Intervertebralimplantate 3 winkelstabil miteinander. Zudem weist die Platte 5 im mittleren und quer verlaufen Bereich 5c zwei Durchgangsöffnungen 6 auf, welche jeweils eine Knochenschraube 25 aufnehmen, die in den mittleren Wirbelkörper 22 eingeschraubt sind und mit denen dadurch die Platte 5 an diesem mittleren Wirbel 22 verankert wird. Auch hier ist eine winkelstabile oder bewegliche Verbindung zwischen Platte 4 und Knochenschraube 25 möglich.

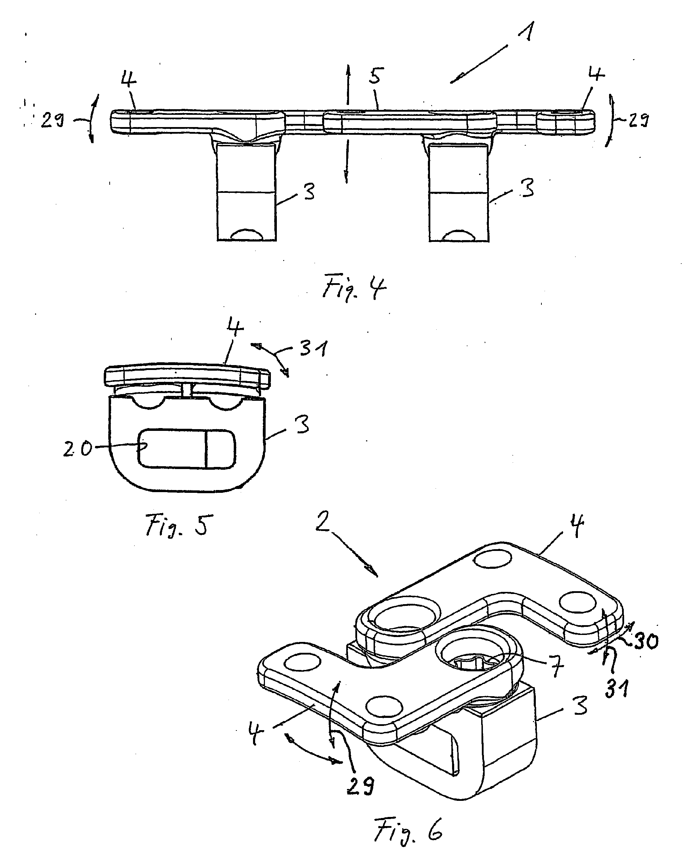

Die beiden Platten 4 und die Platte 5 können gemäss den Fig. 4 und 5 in einer Ebene liegen. Aufgrund der genannten Gelenkverbindungen ist eine solche ebene Anordnung jedoch nicht zwingend. So können die beiden Platten 4 gemäss den Doppelpfeilen 29 der Fig. 4 unabhängig voneinander in einen vergleichsweise grossen Bereich nach oben und nach unten verschwenkt werden. Diese beiden Platten 4 können auch unabhängig voneinander gemäss den Doppelpfeilen 29 bis 31 der Fig. 2, 4, 5 und 6 verschwenkt werden.

Aufgrund der kugelgelenkartigen Verbindungen sind hier aber auch noch andere Bewegungen bzw. Freiheitsgrade möglich. So steht insbesondere die in Fig. 5 mit dem Doppelpfeil 31 angedeutete Schwenkbewegung zur Verfügung. Jede mögliche Position ist durch Festziehen der entsprechenden Kugelkopfschraube 7 fixierbar. Damit ist wie oben erläutert eine optimale Anpassung an die jeweiligen anatomischen Gegebenheiten möglich.

Die Fig. 6 zeigt eine erfindungsgemässe Vorrichtung 2, die lediglich ein Intervertebralimplantat 3 sowie zwei L- bzw. winkelförmige Platten 4 aufweist. Die Platten 4 sind wie bereits oben erläutert jeweils mit einer Kugelkopfschraube 7 am Intervertebralimplantat 3 befestigt. Die Verbindung ist wie oben erläutert kugelgelenkartig. Die beiden Platten 4 können ebenfalls wie oben erläutert vor dem Festziehen der beiden Schrauben 7 bewegt werden. In der Fig. 6 ist lediglich eine der beiden Kugelkopfschrauben 7 gezeigt. Die beiden Platten 4 sind in der gezeigten Ausführung gleich ausgebildet. Die Platten 4 können jedoch auch unterschiedlich sein, beispielsweise unterschiedlich lang sein. Sie müssen nicht zwingend L- bzw. winkelförmig sein, sondern können auch gerade längliche Platten sein. Dies gilt auch für die Platten 4 der Vorrichtung 1. Die Vorrichtung 2 dient zum Verbinden von zwei benachbarten Wirbeln 21 und 22 bzw. 22 und 23. Selbstverständlich können an einer Wirbelsäule 24 mehrere solche Vorrichtungen 2 angebracht werden. Die entsprechenden Intervertebralimplantate 3 sind dann jedoch nur über zwischenliegende Wirbel miteinander verbunden. Eine direkte Verbindung wie bei der Vorrichtung 1 ist damit hier nicht vorhanden.

Die Platten 4 und 5 sowie die Intervertebralimplantate 3 sind vorzugsweise aus einem geeigneten Kunststoff, beispielsweise aus einem faserverstärkten Kunststoff hergestellt. Solche Kunststoffe sind an sich bekannt und haben den wesentlichen Vorteil, dass sie für Röntgenstrahlen durchlässig sind. Mit solchen Materialien können auch sehr stabile Platten 4 und 5 hergestellt werden, die zudem sehr biegesteif sind. Damit ist es möglich, Bewegungen innerhalb der Spondylodese und damit das Risiko einer mitunter folgenschweren sekundären Implantatdislokation zu mindern. Grundsätzlich können die Platten 4 und 5 aber auch aus einem anderen Werkstoff, beispielsweise auch aus Titan hergestellt werden.

Möglich ist auch eine hier nicht gezeigte Vorrichtung mit mehr als zwei Intervertebralimplantaten 3, die mit einer entsprechenden Anzahl von Z-förmigen Platten 5 direkt miteinander verbunden sind. Beispielsweise kann eine solche Vorrichtung drei Intervertebralimplantate 3 aufweisen, die mit zwei Z-förmigen Platten 5 miteinander verbunden sind. An den beiden äusseren Intervertebralimplantaten ist dann jeweils noch eine L-förmige Platte 4 befestigt.

Die Figur 7 zeigt eine Ausführung einer erfindungsgemässen Vorrichtung, welche zwei L-förmigen Platten 4 und eine gerade Platte 32 aufweist. Die lange Platte 32 ist an ihren Enden jeweils mit einer Kugelkopfschraube 7 mit einem Intervertebralimplantat 3 verbunden. Die Verbindungen zu den Intervertebralimplantaten 3 sind Gelenke, die polyaxial beweglich und durch Festziehen der Schrauben 7 fixierbar sind. Die Ausbildung der langen und geraden Platte 32 ergibt sich auch aus der Fig. 9.

Die Figur 8 zeigt zudem eine vergleichsweise kurze Platte 33, die eine Kugelkopfschraube 7 und eine Knochenschraube 25 aufnehmen kann und die insbesondere gemäss Fig. 13 verwendbar ist.

Nachfolgend wird ein Verfahren zur Versteifung eines Abschnittes einer Wirbelsäule mit der erfindungsgemässen Vorrichtung näher erläutert.

Nach einer entsprechenden Freilegung des zu versteifenden Abschnittes der Wirbelsäule wird die befallene Bandscheibe ausgeräumt. Mit einem geeigneten Instrument wird der ausgeräumte Bandscheibenraum erweitert bzw. vergrössert. Nun wird ein passendes Intervertebralimplantat 3 ausgewählt und in den ausgeräumten Bandscheibenraum eingesetzt, wo es zwischen den benachbarten Wirbeln verklemmt. Gegebenenfalls wird eine weitere Bandscheide ausgeräumt und entsprechend ein weiteres Intervertebralimplantat 3 eingesetzt.

Nun werden Abstände zwischen dem Intervertebralimplantat 3 bzw. den Intervertebralimplantaten 3 und geeigneten Positionen für die Knochenschrauben ermittelt. Bei zwei Intervertebralimplantaten 3 wird auch der Abstand zwischen diesen gemessen. Entsprechend diesen Abständen werden passende Platten 4 und gegebenenfalls 5 gewählt und diese lose am Intervertebralimplantat 3 bzw. an den Intervertebralimplantaten fixiert. Die Platten 4 werden so gewählt, dass sie benachbarte Bandscheiben nicht berühren, wie dies in den Figuren 7 und 8 ersichtlich ist. Die lose fixierten Platten 4 und 5 werden an die Wirbel angelegt und mit Knochenschrauben 25 fixiert. Nun werden die Platten 4 und gegebenenfalls 5 durch Festziehen der Schrauben 7 fest mit dem Intervertebralimplantat 3 bzw. Intervertebralimplantaten verbunden.

Die Figuren 10 und 11 zeigen drei Wirbel 21 bis 23, die mit einer erfindungsgemässen Vorrichtung miteinander verbunden sind. Die Platte 5 verbindet wie ersichtlich zwei Intervertebralimplantate 3 miteinander und ist zudem mit Knochenschrauben 25 mit dem Wirbel 22 verbunden. Werden lediglich zwei Wirbel miteinander verbunden, wird die Vorrichtung 2 gemäss Figur 6 verwendet, bei der eine Z-förmige Platte 5 nicht vorgesehen ist.

Die Fig. 11-15 zeigen fünf von den denkbaren Ausführungsmöglichkeiten der erfindungsgemässen Vorrichtung, mit welchen zwei oder mehr Wirbel miteinander verbunden werden können. In den Fig. 11 bis 15 ist die Halswirbelsäule von vorne zu sehen und in der Fig. 10 von der Seite.

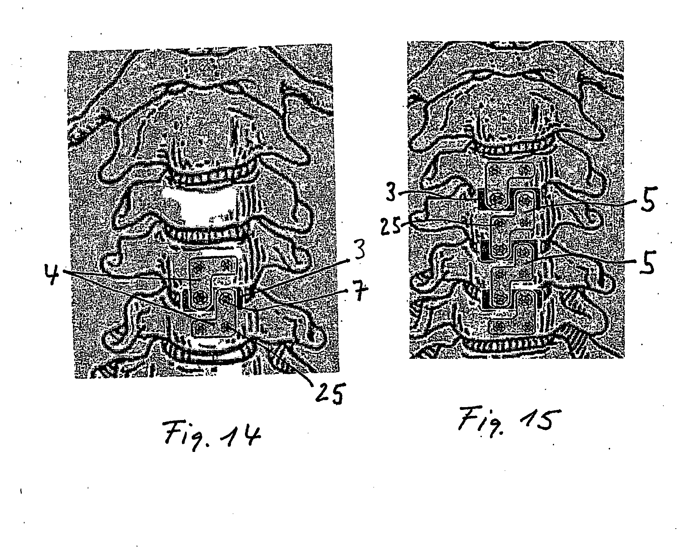

Die Fig. 14 zeigt eine Verbindung von zwei Wirbeln mit Hilfe von zwei L-förmigen Platten 4 und einem Intervertebralimplantat 3. Die L-Platten 4 sind mit je zwei Knochenschrauben 25 an die Wirbelkörper und je einer Kugelkopfschraube 7 an das Intervertebralimplantat 3 befestigt.

Die Fig. 11 zeigt eine Verbindung von drei Wirbeln 21-23. Die Intervertebralimplantate 3 sind durch eine Implantatbrücke direkt miteinander verbunden. Sie besteht in diesem Fall aus einer Z-förmigen Platte 5. Die Z-Platte 5 wird durch zwei Knochenschrauben 25 an den Körper des mittleren Wirbels 22 befestigt.

Die Fig. 10 zeigt eine Ansicht einer Halswirbelsäule von der Seite mit der den Halswirbelkörpern vorne anliegenden erfindungsgemässen Vorrichtung gemäss Fig. 11.

Die Fig. 12 zeigt eine Verbindung von vier Wirbelkörpern, wobei gerade Platten 32 die Implantatbrücke zu den Intervertebralimplantaten 3 bilden. Jede gerade Platte 32 ist mit einer Knochenschraube 25 an den unter der betreffenden Platte 32 liegenden Wirbelkörpern befestigt.

Bei dem Ausführungsbeispiel gemäss Fig. 13 werden drei Intervertebralimplantate 3 nicht direkt durch Implantatbrücken miteinander verbunden, sondern indirekt über den jeweils zwischen zwei Intervertebralimplantaten 3 liegenden Wirbelkörpern, indem kurze Platten 33 an die Intervertebralimplantate 3 und die zwischen ihnen liegenden Wirbelkörper befestigt werden.

Die Fig. 15 zeigt eine Verbindung von vier Wirbeln, wobei wiederum Z-Platten 5 die Implantatbrücken zu Intervertebralimplantaten 3 bilden. Die Z-Platten 5 werden mit je zwei Knochenschrauben 25 an dem unter der jeweiligen Platte befindlichen Wirbelkörper befestigt. Die Verbindung zum oberen und unteren Wirbel erfolgt wie in Fig. 14 beschrieben. Die Verbindung wird in diesem Fall ausschliesslich mit Z-Platten 5 hergestellt.

Ein wesentlicher Vorteil der Erfindung ist, dass das erfindungsgemässe Implantat dank der gelenkigen Cage-Plattenverbindungen einem Sortiment verschieden langer Platten und verschieden grosser (hoher) Intervertebralimplantate den individuell zuweilen sehr unterschiedlichen anatomischen Gegebenheiten, insbesondere der vorderen Oberfläche der Halswirbelsäule, genau angepasst werden kann. Diese kann eben, mehr oder weniger konvex oder sogar konkav oder von Wirbel zu Wirbel wechselnd konvex oder konkav sein. Die Halswirbelsäule kann von vorne gesehen auch etwas krumm oder um die Längsachse etwas verdreht sein. Das alles ist dank der freien Beweglichkeit der Platten ausgleichbar.

Bei der vorliegenden Erfindung geht es insbesondere nicht nur um stabile Verbindungen der Implantate untereinander sowie um ebenso stabile Verbindungen zwischen den Endwirbeln des mit Hilfe der Vorrichtung verblockten Abschnitts der Wirbelsäule, sondern vor allem um die vollständige Anpassbarkeit der Vorrichtung an die jeweiligen anatomischen Gegebenheiten (Krümmungen, Grössenverhältnisse, Unebenheiten). Diese Anpassbarkeit ist besonders an der Halswirbelsäule von grosser Bedeutung, weil die Implantate von der Vorderfläche der Halswirbelsäule nicht vorragen dürfen. Sie dürfen nicht dicker als 2 mm bis höchstens 3 mm sein und müssen den Wirbelkörpern gut anliegen. Anderenfalls besteht die Gefahr, dass eine schwere Komplikation auftreten kann, wenn z.B. der Vorderfläche der Halswirbelsäule eng anliegende Gebilde wie Speiseröhre und Blutgefässe mit der Zeit arrodiert werden.

Unsere Vorrichtung zeichnet sich durch ein niedriges Profil aus, d.h. durch eine niedrige Bauhöhe.

Die Anpassbarkeit der erfindungsgemässen Vorrichtung beruht insbesondere auf
- den blockierbaren Kugelgelenken mit je drei Freiheitsgraden
- einem Sortiment verschieden langer Platten (sowohl L- und Z-Platten)
- einem Sortiment verschieden grosser Intervertebralimplantate.

### Bezugzeichenliste

- 1: Vorrichtung
- 2: Vorrichtung
- 3: Intervertebralimplantat
- 4: Platte
- 4a: Arm
- 4b: Arm
- 5: Platte
- 5a: Arme
- 5b: Arme
- 5c: mittlerer Bereich
- 6: Durchgangsöffnung
- 7: Kugelkopfschraube
- 8: Werkzeugangriff
- 9: Kopf
- 10: Unterseite
- 11: Hals
- 12: Gewindeschaft
- 13: Bohrung
- 14: Fläche
- 15: Fläche
- 17: Durchgangsöffnung
- 18: Gelenkteil
- 19: Durchgangsöffnung
- 21-23: Wirbel
- 24: Halswirbelsäule
- 25: Knochenschraube
- 26: Vorderfläche
- 27: Vertiefung
- 28: Durchgangsöffnung
- 29-31: Doppelpfeile
- 32: gerade Platten
- 33: kurze Platte
- P: mittlerer Punkt

## Patentansprüche

1. Vorrichtung für die Spondylodese und insbesondere für die vordere interkorporelle Spondylodese an der Halswirbelsäule, mit wenigstens einem Intervertebralimplantat (3) und mit wenigstens zwei Platten (4,5), die jeweils mit dem Intervertebralimplantat (3) und einem benachbarten Wirbel (21-23) zu verbinden sind, wobei das Intervertebralimplantat (3) mit den wenigstens zwei im Abstand zueinander angeordneten Platten (4,5) verbunden ist und die beiden Platten (4,5) jeweils an einem Ende mit dem Intervertebralimplantat (3) ein fixierbares Gelenk bilden, **dadurch gekennzeichnet, dass** wenigstens eine der genannten Platten (4,5) an wenigstens einem Ende einen halbkugelförmigen und vorstehenden Gelenkteil (18) aufweist, der einen Durchgang (17) für eine Befestigungsschraube (7) aufweist und der in eine halbkugelförmige Vertiefung (14) des Intervertebralimplantates (3) eingreift, derart, dass ein fixierbares Kugelgelenk gebildet wird.

2. Vorrichtung nach Anspruch 1, **dadurch gekennzeichnet, dass** wenigstens eine der Platten (5, 4) Z-förmig, I-förmig oder L-förmig ausgebildet ist.

3. Vorrichtung nach Anspruch 1 oder 2, **dadurch gekennzeichnet, dass** wenigstens eine der Platten (5) in einem mittleren und quer zur Längsachse der Wirbelsäule (24) verlaufenden Bereich (5c) wenigstens einen Durchgang (6) für eine Knochenschraube (25) aufweist, derart, dass diese Platte (5) mit einem Wirbel (22) verbindbar ist.

4. Vorrichtung nach Anspruch 3, **dadurch gekennzeichnet, dass** der genannte Bereich (5c) zwei im Abstand zueinander angeordnete Durchgänge (6) für jeweils eine Knochenschraube (25) aufweist.

5. Vorrichtung nach Anspruch 1, **dadurch gekennzeichnet, dass** wenigstens zwei Platten (4,5) jeweils über ein polyaxiales Gelenk, insbesondere ein Kugelgelenk mit einem Intervertebralimplantat (3) verbunden sind.

6. Vorrichtung nach einem der Ansprüche 1 - 5, **dadurch gekennzeichnet, dass** wenigstens zwei L-förmige Platten (4) mit einem Intervertebralimplantat (3) verbunden sind.

7. Vorrichtung nach Anspruch 6, **dadurch gekennzeichnet, dass** die beiden Platten (4) jeweils in einem quer zur Längsrichtung der Wirbelsäule (24) verlaufenden Arm (4b) wenigstens zwei im Abstand zueinander angeordnete Durchgänge (19) zur Aufnahme jeweils einer Knochenschraube (25) aufweisen.

8. Vorrichtung nach einem der Ansprüche 1 - 7, **dadurch gekennzeichnet, dass** zwei Intervertebralimplantate (3) mit einer Z-förmigen Platte (5) miteinander verbunden sind.

9. Vorrichtung nach einem der Ansprüche 1 - 8, **dadurch gekennzeichnet, dass** zwei Intervertebralimplantate (3) vorgesehen sind, die mit einer Z-förmigen Platte (5) miteinander verbunden sind und an denen jeweils eine L-förmige oder I-förmige Platte (4) befestigt ist, wobei sämtliche Verbindungen zwischen den Platten (4,5) und den Intervertebralimplantaten (3) als polyaxiale Gelenke, insbesondere Kugelgelenke ausgebildet sind.

10. Vorrichtung nach einem der Ansprüche 1 - 9, **dadurch gekennzeichnet, dass** wenigstens eine Platte (4,5) und/oder ein Intervertebralimplantat (3) aus einem röntgendurchlässigen Werkstoff hergestellt ist bzw. sind.

11. Vorrichtung nach einem der Ansprüche 1 - 10, **dadurch gekennzeichnet, dass** sie für eine Spondylodese der Halswirbelsäule vorgesehen ist.

12. Bausatz zur Herstellung einer Vorrichtung nach einem der Ansprüche 1 - 11, mit wenigstens einem Intervertebralimplantat (3) und wenigstens einer Platte (4,5) zum Verbinden des Intervertebralimplantates (3) mit wenigstens einem Wirbel (21-23), **dadurch gekennzeichnet, dass** wenigstens eine Platte (4) L-förmig und wenigstens eine Platte (5) Z-förmig ausgebildet ist und dass wenigstens ein Intervertebralimplantat (3) im Abstand zueinander zwei Bohrungen (13) zur Aufnahme jeweils einer Befestigungsschraube (7) aufweist und dass wenigstens eine der genannten Platten (4,5) an wenigstens einem Ende einen halbkugelförmigen und vorstehenden Gelenkteil (18) aufweist, der einen Durchgang (17) für eine Befestigungsschraube (7) aufweist und der in eine halbkugelförmige Vertiefung (14) des Intervertebralimplantates (3) eingreift, derart, dass ein fixierbares Kugelgelenk gebildet wird.

13. Bausatz nach Anspruch 12, **dadurch gekennzeichnet, dass** das Intervertebralimplantat (3) und die Platten (4,5) aus einem röntgendurchlässigen Werkstoff hergestellt sind.

14. Bausatz nach Anspruch 12 oder 13, **gekennzeichnet durch** mehrere Knochenschrauben (25) und mehrere Befestigungsschrauben (7)..

15. Bausatz nach Anspruch 14, **dadurch gekennzeichnet, dass** die Befestigungsschrauben Kugelkopfschrauben sind, die einen Schraubenkopf (8) aufweisen, der an seiner Unterseite im Wesentlichen halbkugelförmig ausgebildet ist.

## Claims

1. A device for spondylodesis and in particular for anterior intersomatic spondylodesis of the cervical spine, with at least one intervertebral implant (3) and with at least two plates (4, 5) which in each case are connectable to the intervertebral implant (3) and to an adjacent vertebra (21-23), wherein the intervertebral implant (3) is connected to the at least two plates (4, 5) which are arranged at a distance from one another, one end of each of the two plates (4, 5) forming a fixable joint together with the intervertebral implant (3), **characterized in that** at least one of said plates (4, 5) has, at least at one end, a hemispherical and protruding joint part (18) which has a passage (17) for a locking screw (7) and which engages in a hemispherical depression (14) of the intervertebral implant (3) in such a way that a fixable ball joint is formed.

2. The device as claimed in claim 1, **characterized in that** at least one of the plates (5, 4) has a Z-shaped, I-shaped or L-shaped configuration.

3. The device as claimed in claim 1 or 2, **characterized in that** at least one plate (5), in a central area (5c) extending transversely with respect to the longitudinal axis of the spinal column (24), has at least one passage (6) for a bone screw (25), so that this plate (5) can be connected to a vertebra (22).

4. The device as claimed in claim 3, **characterized in that** said area (5c) has two passages (6) arranged at a distance from one another and each intended for a bone screw (25).

5. The device as claimed in claim 1, **characterized in that** at least said two plates (4, 5) are each connected to an intervertebral implant (3) via a polyaxial joint, in particular a ball joint.

6. The device as claimed in one of claims 1 - 5, **characterized in that** at least two L-shaped plates (4) are connected to an intervertebral implant (3).

7. The device as claimed in claim 6, **characterized in that** the two plates (4) each have, in an arm (4b) extending transversely with respect to the longitudinal direction of the spinal column (24), at least two passages (19) which are arranged at a distance from one another and each receive a bone screw (25).

8. The device as claimed in one of claims 1 - 7, **characterized in that** two intervertebral implants (3) are connected to one another by a Z-shaped plate (5).

9. The device as claimed in one of claims 1 - 8, **characterized in that** two intervertebral implants (3) are provided which are connected to one another by a Z-shaped plate (5) and on each of which an L-shaped or I-shaped plate (4) is secured, all the connections between the plates (4, 5) and the intervertebral implants (3) being designed as polyaxial joints, in particular ball joints.

10. The device as claimed in one of claims 1 - 9, **characterized in that** at least one plate (4, 5) and/or an intervertebral implant (3) is/are made of a material transparent to X-rays.

11. The device as claimed in one of claims 1 - 10, **characterized in that** it is provided for spondylodesis of the cervical spine.

12. A kit for producing the device as claimed in one of claims 1 - 11, with at least one intervertebral implant (3) and with at least one plate (4, 5) for connecting the intervertebral implant (3) to at least one vertebra (21-23), **characterized in that** at least one plate (4) is L-shaped and at least one plate (5) is Z-shaped, and **in that** at least one intervertebral implant (3) has two bores (13) which are arranged at a distance from one another and each receive a locking screw (7), and that at least one of said plates (4, 5) has, at least at one end, a hemispherical and protruding joint part (18) which has a passage (17) for a locking screw (7) and which engages in a hemispherical depression (14) of the intervertebral implant (3) in such a way that a fixable ball joint is formed.

13. The kit as claimed in claim 12, **characterized in that** the intervertebral implant (3) and the plates (4, 5) are made of a material transparent to X-rays.

14. The kit as claimed in claim 12 or 13, **characterized by** a plurality of bone screws (25) and a plurality of locking screws (7).

15. The kit as claimed in claim 14, **characterized in that** the locking screws are ball-head screws having a screw head (8) which is substantially hemispherical on its underside.

## Revendications

1. Dispositif de spondylodèse et en particulier de spondylodèse intercorporelle avant de la colonne vertébrale au niveau du cou, qui présente au moins un implant intervertébral (3) et au moins deux plaques (4, 5) qui sont chacune destinées à être reliées à l'implant intervertébral (3) et à une vertèbre voisine (21-23), l'implant intervertébral (3) étant relié aux deux ou plusieurs plaques (4, 5) disposées à distance l'une de l'autre, les deux plaques (4, 5) formant chacune à une extrémité une articulation qui peut être fixée à l'implant intervertébral (3), **caractérisé en ce qu'**au moins l'une desdites plaques (4, 5) présente à au moins une extrémité une pièce d'articulation (18) de forme hémisphérique et en saillie qui présente un passage (17) pour une vis de fixation (7) et qui s'engage dans un creux hémisphérique (14) de l'implant intervertébral (3) de manière à former une articulation sphérique immobilisable.

2. Dispositif selon la revendication 1, **caractérisé en ce qu'**au moins une des plaques (5, 4) est configurée en forme de Z, de I ou de L.

3. Dispositif selon les revendications 1 ou 2, **caractérisé en ce qu'**au moins une des plaques (5) présente dans une partie centrale (5c) qui s'étend transversalement par rapport à l'axe longitudinal de la colonne vertébrale (24) au moins un passage (6) pour une vis à os (25), de telle sorte que la plaque (5) puisse être reliée à une vertèbre (22).

4. Dispositif selon la revendication 3, **caractérisé en ce que** ladite partie (5c) présente deux passages (6) disposés à distance l'un de l'autre et prévus pour une vis à os (25) respective.

5. Dispositif selon la revendication 1, **caractérisé en ce qu'**au moins deux plaques (4, 5) sont reliées à un implant intervertébral (3) par une articulation polyaxiale et en particulier une articulation sphérique respective.

6. Dispositif selon l'une des revendications 1 à 5, **caractérisé en ce qu'**au moins deux plaques (4) en forme de L sont reliées à un implant intervertébral (3).

7. Dispositif selon la revendication 6, **caractérisé en ce que** les deux plaques (4) présentent chacune dans un bras (4b) qui s'étend transversalement par rapport au sens de la longueur de la colonne vertébrale (24) au moins deux passages (19) disposés à distance l'un de l'autre et qui reprennent chacun une vis à os (25).

8. Dispositif selon l'une des revendications 1 à 7, **caractérisé en ce que** deux implants intervertébraux (3) sont reliés l'un à l'autre par une plaque (5) en forme de Z.

9. Dispositif selon l'une des revendications 1 à 8, **caractérisé en ce que** deux implants intervertébraux (3) qui sont reliés l'un à l'autre par une plaque (5) en forme de Z et à chacun desquels une plaque (4) en forme de L ou en forme de I peut être fixée sont prévus, toutes les liaisons entre les plaques (4, 5) et les implants intervertébraux (3) étant configurées comme des articulations polyaxiales et en particulier comme des articulations sphériques.

10. Dispositif selon l'une des revendications 1 à 9, **caractérisé en ce qu'**au moins une plaque (4, 5) et/ou un implant intervertébral (3) sont réalisés en un matériau transparent aux rayons X.

11. Dispositif selon l'une des revendications 1 à 10, **caractérisé en ce qu'**il est prévu pour la spondylodèse de la colonne vertébrale au niveau du cou.

12. Ensemble destiné à former un dispositif selon l'une des revendications 1 à 11, et qui présente au moins un implant intervertébral (3) et au moins une plaque (4, 5) destinée à relier l'implant intervertébral (3) à au moins une vertèbre (21-23), **caractérisé en ce qu'**au moins une plaque (4) est configurée en forme de L et au moins une plaque (5) est configurée en forme de Z, **en ce qu'**au moins un implant intervertébral (3) présente deux alésages (13) espacés l'un de l'autre qui reprennent chacun une des fixations (7) et **en ce qu'**au moins l'une desdites plaques (4, 5) présente à au moins une extrémité une pièce d'articulation (18) de forme hémisphérique et en saillie qui présente un passage (17) pour une vis de fixation (7) et qui s'engage dans un creux hémisphérique (14) de l'implant intervertébral (3) de manière à former une articulation sphérique immobilisable.

13. Ensemble selon la revendication 12, **caractérisé en ce que** l'implant intervertébral (3) et les plaques (4, 5) sont réalisées en un matériau transparent aux rayons X.

14. Ensemble selon les revendications 12 ou 13, **caractérisé par** plusieurs vis à os (25) et plusieurs vis de fixation (7).

15. Ensemble selon la revendication 14, **caractérisé en ce que** les vis de fixation sont des vis à tête sphérique qui présentent une tête de vis (8) dont le côté inférieur a une forme essentiellement hémisphérique.
